# EUROPEAN PATENT APPLICATION

(11) **EP 3 042 667 A1**
(43) Date of publication of application: **13.07.2016**
(21) Application number: 14842753.7
(22) Date of filing: 27.08.2014
(51) Int. Cl.: A61K 39/00, A61K 39/395, A61P 3/10, C12N 15/09

(54) **DPP-4-TARGETING VACCINE FOR TREATING DIABETES**

(30) Priority: 04.09.2013 JP 2013183390
(71) Applicant: Osaka University, Suita-shi, Osaka 565-0871 (JP)
(72) Inventor: NAKAGAMI, Hironori, Suita-shi Osaka 565-0871 (JP); MORISHITA, Ryuichi, Suita-shi Osaka 565-0871 (JP); KORIYAMA, Hiroshi, Suita-shi Osaka 565-0871 (JP)
(74) Representative: Hoffmann Eitle
(86) International application number: PCT/JP2014/072403
(87) International publication number: WO 2015/033831

(57) **Abstract**

The present invention provides a vaccine for the prophylaxis or treatment of diabetes using a polypeptide containing the amino acid sequence shown by SEQ ID NO: 2 or an amino acid sequence of a non-human mammal, which corresponds to SEQ ID NO: 2, and the like as an immunogen, which induces a neutralizing antibody to DPP-4, and a prophylactic or therapeutic agent for diabetes, which contains a DDP-4 neutralizing antibody that recognizes a partial amino acid sequence of the aforementioned DDP-4.

SEQUENCE LISTING

<110>  Osaka University

<120>  Vaccine for treating diabetes targeting DPP-4

<130>  092210

<150>  JP 2013-183390
<151>  2013-09-04

<160>  6

<170>  PatentIn version 3.5

<210>  1
<211>  27
<212>  DNA
<213>  Homo sapiens

<400>  1
gagaacagta catttgatga gtttgga                                          27


<210>  2
<211>  9
<212>  PRT
<213>  Homo sapiens

<400>  2

Glu Asn Ser Thr Phe Asp Glu Phe Gly
1               5


<210>  3
<211>  27
<212>  DNA
<213>  Mus musculus

<400>  3
gagaacagta cctttgaaag ctttgga                                          27


<210>  4
<211>  9
<212>  PRT
<213>  Mus musculus

<400>  4

Glu Asn Ser Thr Phe Glu Ser Phe Gly
1               5


<210>  5
<211>  12
<212>  PRT
<213>  Mus musculus

<400>  5

Ser Lys Asp Glu Ala Ala Ala Asp Ser Arg Arg Thr

1                   5                        10

<210>  6
<211>  10
<212>  PRT
<213>  Mus musculus

<400>  6

Lys Ser Thr Phe Arg Val Lys Ser Tyr Ser
1                   5                        10

## Description

### Technical Field

The present invention relates to a vaccine for the prophylaxis or treatment of diabetes, comprising a particular partial amino acid sequence of DPP-4(dipeptidyl peptidase 4) as an immunogen, and a prophylactic or therapeutic agent for diabetes, comprising a DPP-4 neutralizing antibody that recognizes the aforementioned partial amino acid sequence of DPP-4.

### Background Art

Diabetes is a metabolic disease wherein blood glucose increases more than in healthy individuals due to insufficient quantity or insufficient action of insulin in the body, which in turn causes microangiopathy in the kidney, retina, nerves and the like and macroangiopathy such as arteriosclerosis and the like to markedly impair healthy life. Heretofore, hypoglycemic agents such as insulin, insulin secretagogue, insulin sensitizer, α-glucosidase inhibitor and the like have been widely applied as a clinical treatment method. Although usefulness of these hypoglycemic agents has been acknowledged, each of them has many problems. For example, insulin has a risk of causing hypoglycemia when administered by an inappropriate method or dose. In diabetic patients showing markedly decreased insulin secretion capacity of the pancreas, the effectiveness of insulin secretagogues and insulin sensitizers decreases. In diabetic patients having remarkable insulin resistance, the effectiveness of insulin and insulin secretagogue decreases.

It is known that plural factors are involved in the etiology of diabetes. As one of them, GLP-1 (glucagon-like peptide-1), a known incretin as gastrointestinal tract hormone, is involved in glucose metabolism. GLP-1 not only stimulates insulin secretion but also stimulates skeletal muscle, adipose tissue, and liver to improve insulin sensitivity, and can control blood glucose level. However, it is rapidly degraded by DPP-4, a serine protease (non-patent document 1). For treating diabetes, therefore, drug discovery targeting DPP-4 has been investigated and some DPP-4 inhibitors have been marketed to date. However, since the administration frequency and dose tend to increase, thus problematically placing a large economical burden on the patients.

### [Document List]

### [non-patent document]

non-patent document 1: J Biol Chem 1996; 271(38): 23222-23229

### SUMMARY OF THE INVENTION

### Problems to be Solved by the Invention

The present invention aims to provide a vaccine for the prophylaxis or treatment of diabetes comprising a particular partial amino acid sequence of DPP-4, and a prophylactic or therapeutic agent for diabetes, comprising a DPP-4 neutralizing antibody that recognizes the aforementioned partial amino acid sequence of DPP-4, which are to be used for a method for the prophylaxis or treatment of diabetes, which is superior to conventional DPP-4 inhibitors in the dose and administration frequency.

### Means of Solving the Problems

The present inventors inferred plural amino acid moieties important for the binding to and cleavage of GLP-1 from the information of the three-dimensional structure of DPP-4, and designed the moieties as antigen candidates capable of inducing a neutralization activity on DPP-4. The aforementioned synthesized plural antigen candidates were conjugated with KLH and administered to mouse together with a Freund's adjuvant. As a result, two kinds of antigen candidates showing a significant increase in the antibody were specified. The mouse administered with the two kinds of antigen candidates was further fed with a high-fat diet. As a result, the mouse immunized with one kind of antigen candidate showed a high DPP-4 activity inhibitory rate, and improvement in insulin secretion and glucose tolerance, as compared to the control. An increase in the antibody titer induced in mouse administered with the antigen candidate 3 times was sustained for about 3 months. Readministration of the antigen candidate thereafter increased the antibody titer again with ease by a booster effect.

Based on these findings, the present inventors have conducted further studies and completed the present invention.

That is, the present invention provides
[1] a vaccine for the prophylaxis or treatment of diabetes, comprising any of the following substances (1) - (3):
   (1) a polypeptide comprising the amino acid sequence shown by SEQ ID NO: 2 or an amino acid sequence of a non-human mammal, which corresponds to SEQ ID NO: 2;
   (2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added; and
   (3) an expression vector capable of expressing the polypeptide of the above-mentioned (1) or (2);
[2] the vaccine of [1], comprising a carrier protein;
[3] the vaccine of [1] or [2], which comprises an adjuvant;
[4] a prophylactic or therapeutic agent for diabetes, comprising an antibody recognizing the polypeptide of the following (1) or (2), and inhibiting the function of DPP-4:
   (1) a polypeptide comprising the amino acid sequence shown by SEQ ID NO: 2 or an amino acid sequence of a non-human mammal, which corresponds to SEQ ID NO: 2; and
   (2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added;
[5] a method for the prophylaxis or treatment of diabetes, comprising administering an effective amount of any of the following substances (1) - (3) to a subject:
   (1) a polypeptide comprising the amino acid sequence shown by SEQ ID NO: 2 or an amino acid sequence of a non-human mammal, which corresponds to SEQ ID NO: 2;
   (2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added; and
   (3) an expression vector capable of expressing the polypeptide of the above-mentioned (1) or (2);
[6] the method of [5], comprising administering a carrier protein;
[7] the method of [5] or [6], comprising administering an adjuvant;
[8] a method for the prophylaxis or treatment of diabetes, comprising administering an effective amount of an antibody recognizing the polypeptide of the following (1) or (2), and inhibiting the function of DPP-4 to a subject:
   (1) a polypeptide comprising the amino acid sequence shown by SEQ ID NO: 2 or an amino acid sequence of a non-human mammal, which corresponds to SEQ ID NO: 2; and
   (2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added;
[9] any of the following substances (1) - (3) for use for a method for the prophylaxis or treatment of diabetes:
   (1) a polypeptide comprising the amino acid sequence shown by SEQ ID NO: 2 or an amino acid sequence of a non-human mammal, which corresponds to SEQ ID NO: 2;
   (2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added; and
   (3) an expression vector capable of expressing the polypeptide of the above-mentioned (1) or (2);
[10] the substance of [9], comprising a carrier protein;
[11] the substance of [9] or [10], comprising an adjuvant;
[12] an antibody recognizing the polypeptide of the following (1) or (2), and inhibiting the function of DPP-4, which is for use for a method for the prophylaxis or treatment of diabetes:
   (1) a polypeptide comprising the amino acid sequence shown by SEQ ID NO: 2 or an amino acid sequence of a non-human mammal, which corresponds to SEQ ID NO: 2; and
   (2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added;
[13] a polypeptide consisting of the amino acid sequence shown by SEQ ID NO: 2.

### Effect of the Invention

Using the partial amino acid sequence of DPP-4 of the present invention as a vaccine, a neutralizing antibody that inhibits DPP-4 activity can be induced, degradation of GLP-1 can be inhibited by the antibody, and insulin secretion can be improved. Using a neutralizing antibody recognizing a partial amino acid sequence of DPP-4, moreover, the above-mentioned effect can be obtained directly. Furthermore, since the half-life of the neutralizing antibody is longer than that of the conventional DPP-4 inhibitors, the administration frequency of the vaccine and antibody to patients can be expected to be decreased than conventional DPP-4 inhibitors.

### Brief Description of the Drawings

Fig. 1 provides graphs showing:
   A: antibody titer to DPP-4 in mouse immunized with E1, E2, E3 or KLH vaccine, *:P<0.05, **:P<0.01, ***:P<0.001. B: binding of antibody derived from serum of E1 or E3 vaccine group to recombinant DPP-4 protein.
Fig. 2 provides graphs showing:
   A: inhibitory rate of anti-DPP-4 antibody induced with E1 or E3 vaccine on DPP-4 function in the plasma of mouse on day 56 of immunization with E1 or E3 vaccine, *:P<0.05. B: in vitro inhibitory rate of the serum of mouse on day 56 of immunization with E1 vaccine (20 µg/mouse) or E3 vaccine (20 µg/mouse) on DPP-4 function, *:P<0.05. C: GLP-1 level of plasma after glucose administration in mouse immunized with KLH, E1 or E3 vaccine, *:P<0.05.

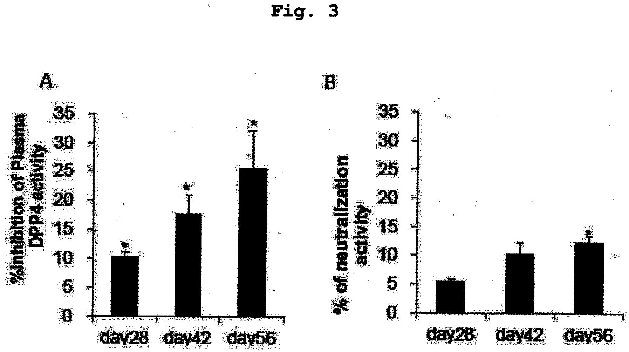

Fig. 3 provides graphs showing:
   A: inhibitory rate of anti-DPP-4 antibody induced with E3 vaccine on DPP-4 function in the plasma of mouse on days 28, 42, 56 of immunization with E3 vaccine, *:P<0.05. B: in vitro inhibitory rate of the serum of mouse on days 28, 42, 56 of immunization with E3 vaccine (20 µg/mouse) on DPP-4 function, *:P<0.05.

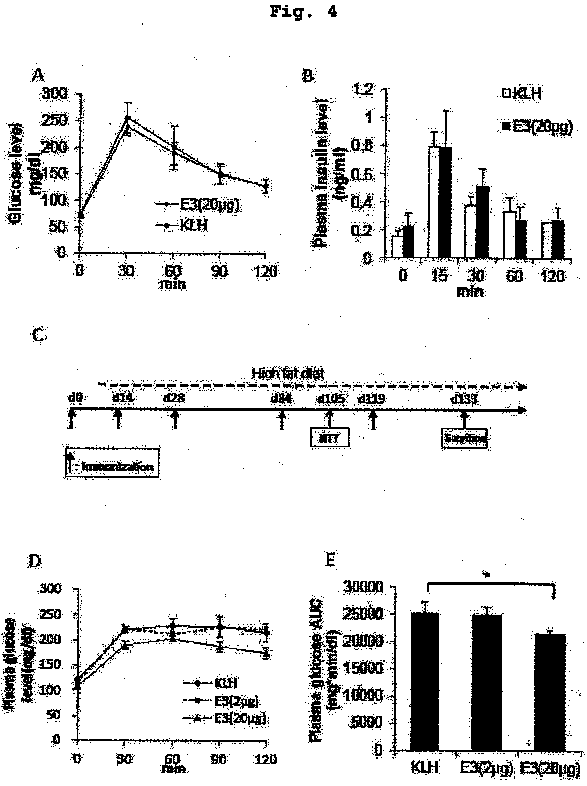

Fig. 4 provides graphs showing:
   A: glucose level of mouse immunized with E3 or KLH vaccine after meal challenge, under normal diet conditions. B: insulin level of mouse immunized with E3 or KLH vaccine after meal challenge, under normal diet conditions. C: implementation plans of oral meal tolerance test (MTT) in mouse immunized with E3 or KLH vaccine, under high-fat diet conditions. D: glucose level of mouse immunized with E3 or KLH vaccine after meal challenge, under high-fat diet conditions. E: total glucose level, for 0 - 120 min after meal challenge, of mouse immunized with E3 or KLH vaccine, under high-fat diet conditions, *:P<0.05.

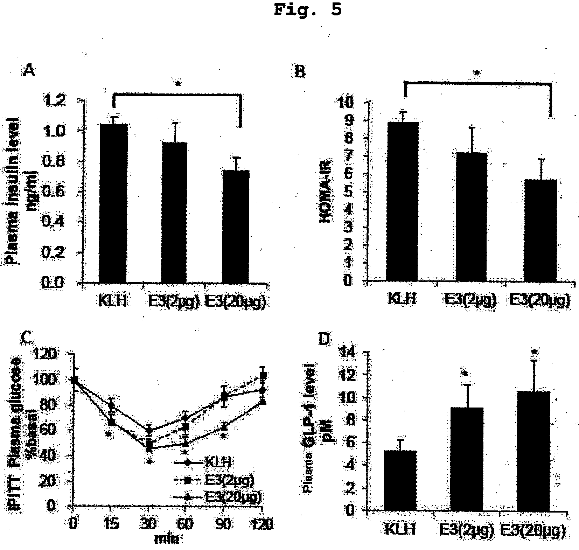

Fig. 5 provides graphs showing:
   A: insulin level of mouse immunized with E3 or KLH vaccine, under high-fat diet conditions, *:P<0.05. B: HOMA-IR of mouse immunized with E3 or KLH vaccine, under high-fat diet conditions, *:P<0.05. C: glucose level of mouse immunized with E3 or KLH vaccine and after intraperitoneal insulin administration, under high-fat diet conditions. The glucose level at the time of insulin administration was taken as 100%, *:P<0.05. D: GLP-1 level of mouse immunized with E3 or KLH vaccine and after intraperitoneal insulin administration, under high-fat diet conditions, *:P<0.05.

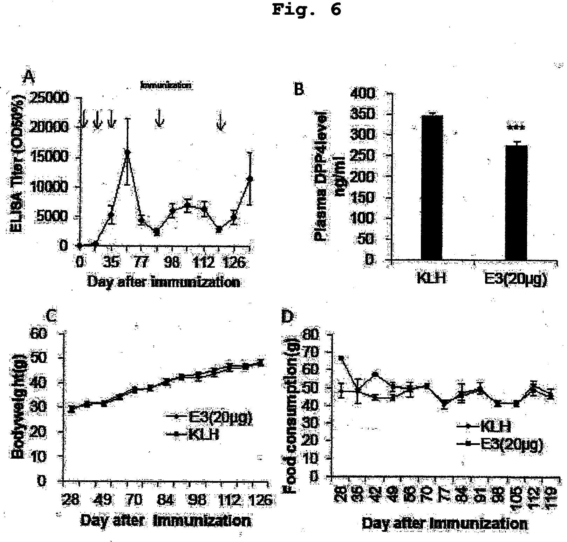

Fig. 6 provides graphs showing:
   A: time course changes of antibody titer of mouse immunized with E3 vaccine. B: DPP-4 level of mouse immunized with E3 or KLH vaccine, under high-fat diet conditions, ***:P<0.001. C: body weight of mouse immunized with E3 or KLH vaccine, under high-fat diet conditions. D: diet consumption amount of mouse immunized with E3 or KLH vaccine, under high-fat diet conditions.

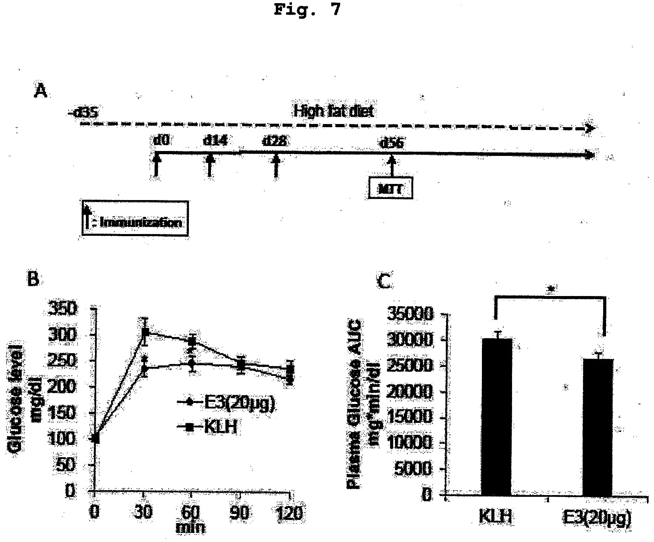

Fig. 7 provides graphs showing:
   A: implementation plans of oral meal tolerance test (MTT) in diabetic model mouse immunized with E3 or KLH vaccine, under high-fat diet conditions. B: glucose level of diabetic model mouse immunized with E3 or KLH vaccine and after meal challenge, under high-fat diet conditions, *:P<0.05. C: total glucose level, for 0 - 120 min after meal challenge, of diabetic model mouse immunized with E3 or KLH vaccine, under high-fat diet conditions, *:P<0.05.

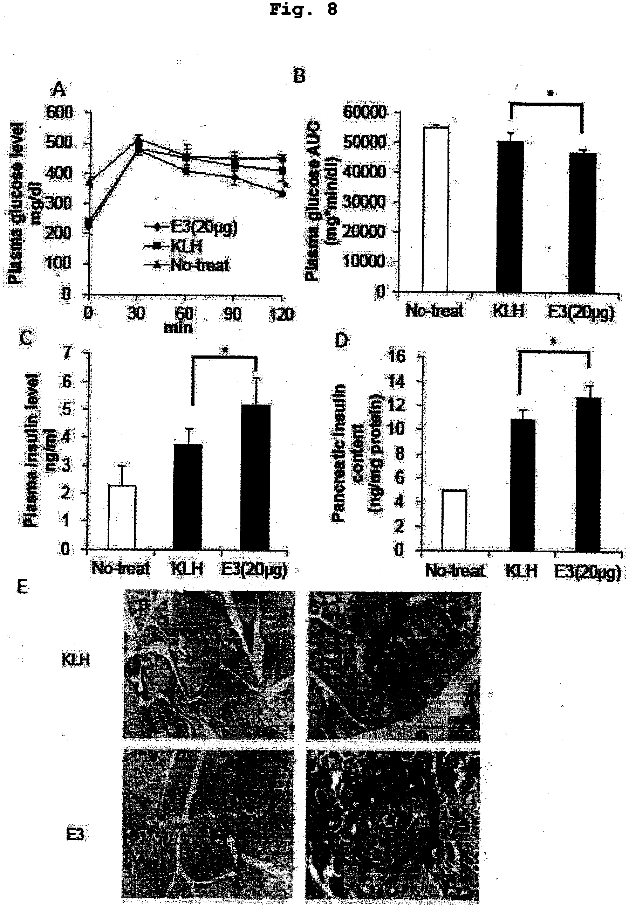

Fig. 8 provides graphs showing:
   A: glucose level after meal challenge of non-immunized db/db mouse, db/db mouse immunized with E3 or KLH vaccine, *:P<0.05.
   B: total glucose level, for 0 - 120 min after meal challenge, of non-immunized db/db mouse, mouse immunized with E3 or KLH vaccine, *:P<0.05. C: insulin level after meal challenge of non-immunized db/db mouse, db/db mouse immunized with E3 or KLH vaccine, *:P<0.05. D: pancreatic insulin content after meal challenge of non-immunized db/db mouse, db/db mouse immunized with E3 or KLH vaccine, *:P<0.05. E: microscopic images of immunohistostained sections derived from the pancreas of db/db mouse immunized with E3 or KLH vaccine and after meal challenge.

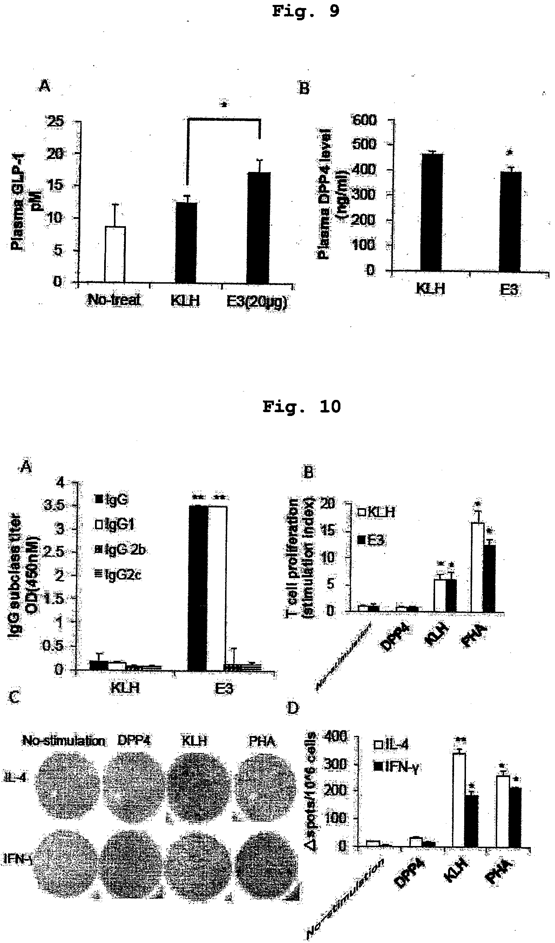

Fig. 9 provides graphs showing:
   A: GLP-1 level of non-immunized db/db mouse, db/db mouse immunized with E3 or KLH vaccine, *:P<0.05. B: DPP-4 level of db/db mouse immunized with E3 or KLH vaccine, *:P<0.05.
Fig. 10 provides graphs showing:
   A: antibody titer of each IgG subclass induced in mouse immunized with E3 or KLH vaccine, **:P<0.01. B: T cell proliferation assay of splenocytes derived from mouse immunized with E3 or KLH vaccine. C: ELISPOT assay of splenocytes derived from mouse immunized with E3 vaccine. D: the number of colored spots in ELISPOT assay of splenocytes derived from mouse immunized with E3 vaccine.

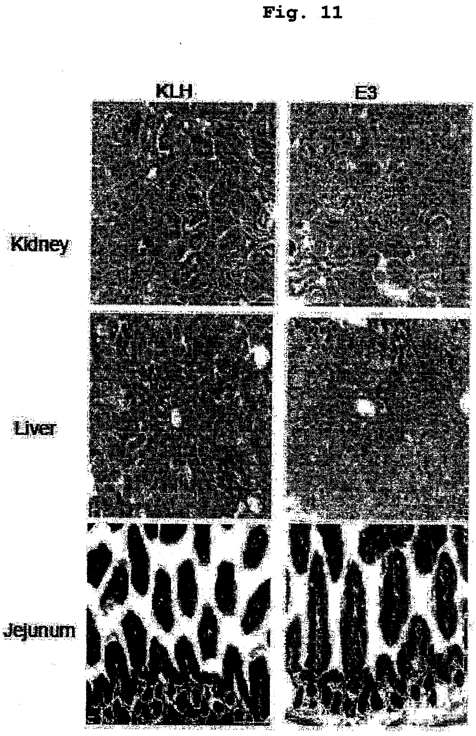

Fig. 11 shows microscopic images of Masson trichromestained sections derived from the kidney, liver and jejunum of mouse immunized with E3 or KLH vaccine.

### Description of Embodiments

### 1. Vaccine for the prophylaxis or treatment of diabetes

The present invention provides a vaccine for the prophylaxis or treatment of diabetes, comprising any of the following substances (1) - (3):
(1) a polypeptide comprising the amino acid sequence shown by SEQ ID NO: 2 or an amino acid sequence of a non-human mammal, which corresponds to SEQ ID NO: 2;
(2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added; and
(3) an expression vector capable of expressing the polypeptide of the above-mentioned (1) or (2).

In the present specification, diabetes includes, in addition to diabetes (type 1 diabetes, type 2 diabetes), diseases involving diabetes. Examples of the diseases involving diabetes include diabetic neuropathy, diabetic nephropathy, diabetic retinopathy, hyperinsulinemia, obesity and the like.

The subject of administration of the vaccine of the present invention may be any mammal, which is a mammal who has developed diabetes or having a risk of developing diabetes. Examples of the mammal include experiment animals such as rodents (e.g., mouse, rat, hamster, guinea pig and the like), rabbit and the like, pets such as dog, cat and the like, domestic animals such as bovine, swine, goat, horse, sheep and the like, primates such as human, monkey, orangutan, chimpanzee and the like, and the like, and human is particularly preferable. The subject of administration may or may not be undergoing treatments for diabetes. When the vaccine of the present invention is administered, substances contained in the vaccine are preferably those derived from the subject of administration (that is, when administered to human, the vaccine is a substance derived from human, and when administered to mouse, the vaccine is a substance derived from mouse).

The substance contained in the vaccine of the present invention is a substance selected from the group consisting of,
(1) a polypeptide comprising the amino acid sequence shown by SEQ ID NO: 2 or an amino acid sequence of a non-human mammal, which corresponds to SEQ ID NO: 2;
(2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added; and
(3) an expression vector capable of expressing the polypeptide of the above-mentioned (1) or (2).

The polypeptide of the above-mentioned (1) (hereinafter the polypeptide of the present invention, including the polypeptide of the above-mentioned (2)) contained in the vaccine of the present invention is a partial sequence of the amino acid sequence of DPP-4 (dipeptidyl peptidase 4). DPP-4 is a known gene and the nucleotide sequence thereof and the amino acid sequence thereof are also known. For example, a specific amino acid sequence contained in the polypeptide of the above-mentioned (1) is, for example, the nucleotide sequence shown by SEQ ID NO: 2. The partial sequence is encoded by, for example, the nucleotide sequence shown by SEQ ID NO: 1. Furthermore, as a preferable amino acid sequence to be contained in the polypeptide of the above-mentioned (1), a polypeptide containing an amino acid sequence of a non-human mammal, which corresponds to SEQ ID NO: 2, can be mentioned. An amino acid sequence of a non-human mammal, which corresponds to SEQ ID NO: 2, can be obtained easily by designing appropriate primers and probes by utilizing the information of the amino acid sequence disclosed in SEQ ID NO: 2 in the present specification, and known sequence database, and using a general genetic engineering method such as RT-PCR, plaque hybridization and the like. For example, a partial sequence of the amino acid sequence of mouse DPP-4, which corresponds to SEQ ID NO: 2 which is a partial sequence of human DPP-4, is an amino acid sequence shown by SEQ ID NO: 4, and the partial sequence is encoded by, for example, the nucleotide sequence shown by SEQ ID NO: 3. As used herein, non-human mammal refers to the above-mentioned mammals excluding human.

The polypeptide of the above-mentioned (1) to be contained in the vaccine of the present invention is preferably a polypeptide consisting of the amino acid sequence shown by SEQ ID NO: 2 or an amino acid sequence of a non-human mammal, which corresponds to SEQ ID NO: 2.

The polypeptide of the above-mentioned (2) to be contained in the vaccine of the present invention is a partial sequence of the amino acid sequence of DPP-4 wherein 1 or several (preferably 1 - several (2 - 5)) amino acids are deleted, substituted, inserted or added. Such polypeptide includes, in the case of human, the amino acid sequence shown by SEQ ID NO: 2 wherein 1 or several (preferably 1 - several (2 - 5)) amino acids are deleted, substituted, inserted or added. Examples of the amino acid sequence include (1) the amino acid sequence shown by SEQ ID NO: 2 wherein 1 or several (preferably 1 - several (2 - 5)) amino acids are deleted, (2) the amino acid sequence shown by SEQ ID NO: 2 added with 1 or several (preferably 1 - several (2 - 5)) amino acids, (3) the amino acid sequence shown by SEQ ID NO: 2 inserted with 1 or several (preferably 1 - several (2 - 5)) amino acids, (4) the amino acid sequence shown by SEQ ID NO: 2 wherein 1 or several (preferably 1 - several (2 - 5)) amino acids are substituted by other amino acids, and (5) an amino acid sequence having the above-mentioned mutations (1) - (4) in combination (in this case, the total of the mutated amino acids is 1 or several (preferably 1 - several (2 - 5))). In addition, the amino acid sequence to be contained in the polypeptide of above-mentioned (2), an amino acid sequence of a non-human mammal, which corresponds to SEQ ID NO: 2 and in which 1 or several (preferably 1 - several (2 - 5)) amino acids are deleted, substituted, inserted or added can be preferably mentioned. Such polypeptide includes, in the case of mouse, the amino acid sequence shown by SEQ ID NO: 4 wherein 1 or several (preferably 1 - several (2 - 5)) amino acids are deleted, substituted, inserted or added. Examples of the amino acid sequence include (1) the amino acid sequence shown by SEQ ID NO: 4 wherein 1 or several (preferably 1 - several (2 - 5)) amino acids are deleted, (2) the amino acid sequence shown by SEQ ID NO: 4 added with 1 or several (preferably 1 - several (2 - 5)) amino acids, (3) the amino acid sequence shown by SEQ ID NO: 4 inserted with 1 or several (preferably 1 - several (2 - 5)) amino acids, (4) the amino acid sequence shown by SEQ ID NO: 4 wherein 1 or several (preferably 1 - several (2 - 5)) amino acids are substituted by other amino acids, and (5) an amino acid sequence having the above-mentioned mutations (1) - (4) in combination (in this case, the total of the mutated amino acids is 1 or several (preferably 1 - several (2 - 5))).

Examples of the "substitution of amino acid residue" include preservative amino acid substitution. The preservative amino acid substitution refers to substitution of a particular amino acid with an amino acid having a side chain having properties similar to those of the side chain of the amino acid. Specifically, in the preservative amino acid substitution, a particular amino acid is substituted by other amino acid in the group to which the amino acid belongs. The group of amino acids having a side chain with similar properties is known in the pertinent field. Examples of the group of such amino acids include amino acids having a basic side chain (e.g., lysine, arginine, histidine), amino acids having an acidic side chain (e.g., aspartic acid, glutamic acid), amino acids having a neutral side chain (e.g., glycine, asparagine, glutamine, serine, threonine, tyrosine, cysteine, alanine, valine, leucine, isoleucine, proline, phenylalanine, methionine, tryptophan). The amino acids having a neutral side chain can be further divided into amino acid having a polar side chain (e.g., glycine, asparagine, glutamine, serine, threonine, tyrosine, cysteine), and amino acid having a nonpolar side chain (e.g., alanine, valine, leucine, isoleucine, proline, phenylalanine, methionine, tryptophan). As other group, for example, amino acids having an aromatic side chain (e.g., phenylalanine, tryptophan, tyrosine), amino acids having a side chain containing a hydroxyl group (alcoholic hydroxyl group, phenolic hydroxyl group) (e.g., serine, threonine, tyrosine) and the like can also be mentioned.

Examples of the "deletion of amino acid residue" include selection and deletion of any amino acid residue of the amino acid sequence shown by SEQ ID NO: 2.

Examples of the "insertion of amino acid residue" or "addition of amino acid residue" include insertion or addition of amino acid residue inside or to the N-terminal side or C-terminal side of the amino acid sequence shown by SEQ ID NO: 2. To enhance water solubility of peptide, 1 or 2 residues of arginine (Arg) or lysine (Lys), which are basic amino acids, may be added to the N-terminal side or C-terminal side of the amino acid sequence.

The polypeptide of the above-mentioned (2) to be contained in the vaccine of the present invention is preferably a polypeptide consisting of the amino acid sequence shown by SEQ ID NO: 2, or an amino acid sequence of a non-human mammal, which corresponds to SEQ ID NO: 2, wherein one or several amino acid residues are substituted, deleted, inserted or added.

The polypeptide of the present invention may contain an addition amino acid. Addition of such amino acid is acceptable as long as the polypeptide induces a specific immune reaction to DPP-4. While the amino acid sequence to be added is not particularly limited, for example, a tag that facilitates detection, purification and the like of the polypeptide can be mentioned. Examples of the tag include Flag tag, histidine tag, c-Myc tag, HA tag, AU1 tag, GST tag, MBP tag, fluorescence protein tag (e.g., GFP, YFP, RFP, CFP, BFP etc.), immunoglobulin Fc tag and the like. The position at which the amino acid sequence is added is N-terminal and/or C-terminal of the polypeptide of the present invention.

While the amino acid to be used for the polypeptide of the present invention encompasses an L form, D form and DL form, it is generally preferably an L form. These polypeptides can be synthesized by a general polypeptide synthesis method and subjected to the present invention. In the present invention, the production method, synthesis method, supply method and the like are not particularly limited.

In the expression vector of the above-mentioned (3), a polynucleotide encoding the polypeptide of the aforementioned (1) or (2) (DNA or RNA, preferably DNA) is functionally linked to the downstream of a promoter capable of exhibiting the promoter activity in the cells of a mammal to be the subject of administration. That is, the expression vector of (3) can express the polypeptide of (1) or (2) as a transcription product under the control of the promoter. By the administration of the expression vector of (3) to a mammal, the polypeptide of (1) or (2) is produced in the body of the mammal, whereby a specific immune reaction to the polypeptide of (1) or (2) is induced in the mammal.

The promoter to be used is not particularly limited as long as it can function in the cells of a mammal to be the subject of administration. As the promoter, polI type promoter, polII type promoter, polIII type promoter and the like can be used. Specifically, initial promoter derived from SV40, virus promoters such as cytomegalovirus LTR and the like, constituent protein gene promoter of a mammal such as β-actin gene promoter and the like, and the like are used.

The expression vector of the above-mentioned (3) preferably contains a transcription termination signal, i.e., terminator region, at the downstream of the polynucleotide encoding the polypeptide of the aforementioned (1) or (2). Furthermore, it can further contain a selection marker gene (gene imparting resistant to drugs such as tetracycline, ampicillin, kanamycin and the like, gene complementing auxotrophic mutation etc.) for the selection of a transformed cell.

While the kind of the vector to be used as an expression vector in the present invention is not particularly limited, a vector preferable for the administration to a mammal such as human and the like includes virus vector, plasmid vector and the like. Examples of the virus vector include retrovirus, adenovirus, adeno-associated virus and the like. In consideration of the production, easy handleability and economic efficiency, a plasmid vector is preferably used.

The vaccine of the present invention can be provided as a pharmaceutical composition containing any carrier, for example, a pharmaceutically acceptable carrier, in addition to the polypeptide of the above-mentioned (1) or (2) or the expression vector of (3).

Examples of the pharmaceutically acceptable carrier include, but are not limited to, excipients such as sucrose, starch and the like, binders such as cellulose, methylcellulose and the like, disintegrants such as starch, carboxymethylcellulose and the like, lubricants such as magnesium stearate, aerogel and the like, aromatics such as citric acid, menthol and the like, preservatives such as sodium benzoate, sodium bisulfite and the like, stabilizers such as citric acid, sodium citrate and the like, suspending agents such as methylcellulose, polyvinylpyrrolidone and the like, dispersing agents such as surfactant and the like, diluents such as water, saline and the like, base-wax and the like.

When the vaccine of the present invention is the expression vector of the above-mentioned (3), to promote introduction of the expression vector into the cell, the vaccine of the present invention can further contain a reagent for the introduction of the nucleic acid. When a virus vector is used as the expression vector, RetroNectin, fibronectin, polybrene and the like can be used as the transgene reagent. When a plasmid vector is used as an expression vector, cationic lipids such as Lipofectin, lipofectamine, DOGS (Transfectam), DOPE, DOTAP, DDAB, DHDEAB, HDEAB, polybrene, or poly(ethyleneimine) (PEI) and the like can be used.

The vaccine of the present invention preferably further contains a carrier protein to increase the immunogenicity of the polypeptide of the above-mentioned (1) or (2) or the polypeptide encoded by the expression vector of (3). A carrier protein is generally a substance that binds to a molecule having no immunogenicity due to its small molecular weight and imparts the immunogenicity, and is known in the Technical Field. Preferable examples of the carrier protein include bovine serum albumin (BSA), rabbit serum albumin (RSA), ovalbumin (OVA), keyhole-limpet hemocyanin (KLH), thyroglobulin (TG), immunoglobulin and the like. A particularly preferable carrier protein is keyhole-limpet hemocyanin (KLH). In the case of the above-mentioned expression vector of (3), a polynucleotide encoding the carrier protein may be linked to a polynucleotide encoding the polypeptide of the above-mentioned (1) or (2).

Also, the vaccine of the present invention preferably further contains a pharmaceutically acceptable adjuvant compatible with the active ingredient. Adjuvant is generally a substance that non-specifically potentiates an immune response of the host, and a number of various adjuvants are known in the technical field. Examples of the adjuvant include, but are not limited to, the following: complete Freund's adjuvant, incomplete Freund's adjuvant, aluminum hydroxide, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-sn-glycerol-3-hydroxyphosphoryloxy)-ethylamine (MTP-PE), Quill A (registered trade mark), lysolecithin, saponin derivative, pluronic polyol, montanide ISA-50 (Seppic, Paris, France), Bayol (registered trade mark) and Markol (registered trade mark).

The vaccine of the present invention can be administered orally or parenterally to a mammal. Since polypeptide and expression vector are decomposed in the stomach, parenteral administration is preferable. A preparation preferable for oral administration includes liquid, capsule, sachet, tablet, suspension, emulsion and the like. A preparation preferable for parenteral administration (e.g., subcutaneous injection, muscular injection, topical injection, intraperitoneal administration and the like) includes aqueous and non-aqueous isotonic, aseptic injection liquid, which optionally contains antioxidant, buffer, bacteriostatic agent, isotonicity agent and the like. Also, an aqueous and non-aqueous aseptic suspending agent can be mentioned, and the agent optionally contains suspending agent, solubilizer, thickener, stabilizer, preservative and the like. Such preparation can be sealed in a unit dose or plural dose container such as ampoule or vial. In addition, the active ingredient and pharmaceutically acceptable carriers may be freeze-dried, and preserved in a form only requiring dissolution or suspending in a suitable aseptic vehicle immediately before use.

The content of the active ingredient in a pharmaceutical composition is generally about 0.1 - 100 wt%, preferably about 1 - 99 wt%, more preferably about 10 - 90 wt%, of the whole pharmaceutical composition.

While the dose of the vaccine of the present invention varies depending on the administration subject, administration method, administration form and the like, when the active ingredient is the polypeptide of the above-mentioned (1) or (2), 1 µg 1000 µg, preferably 20 µg - 100 µg, of the polypeptide is generally administered per dose to an adult 2 or 3 times for generally 4 weeks to 12 weeks. When the antibody titer falls, addition administration is performed once each time. When the active ingredient is the expression vector of the above-mentioned (3), 1 µg - 1000 µg, preferably 20 µg - 100 µg, of the polypeptide is generally administered per dose to an adult 2 or 3 times for generally 4 weeks to 12 weeks. When the antibody titer falls, addition administration is performed once each time.

By the administration of the vaccine of the present invention to a mammal, immune responses specific to DPP-4 (specific antibody production, growth of specific T cells etc.) are induced, the mammal acquires a neutralizing antibody to DPP-4, and the function of DPP-4 is inhibited, whereby degradation of GLP-1 is suppressed to consequently provide a prophylactic or therapeutic effect for diabetes.

In addition, the present invention provides a kit composed of one or more containers containing one or more components of the vaccine of the present invention. Diabetes can also be prevented, or the symptoms thereof can be treated or alleviated using the kit of the present invention.

### 2. DPP-4 neutralizing antibody for prophylaxis or treatment of diabetes

The present invention also provides a prophylactic or therapeutic agent for diabetes, which comprises an antibody recognizing the polypeptide of the following (1) or (2), and inhibiting the function of DPP-4 (the prophylactic or therapeutic agent of the present invention):
(1) a polypeptide comprising the amino acid sequence shown by SEQ ID NO: 2 or an amino acid sequence of a non-human mammal, which corresponds to SEQ ID NO: 2;
(2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added.

The polypeptide of the aforementioned (1) is preferably a polypeptide consisting of the amino acid sequence shown by SEQ ID NO: 2 or an amino acid sequence of a non-human mammal, which corresponds to SEQ ID NO: 2. The polypeptide of the aforementioned (2) is preferably a polypeptide consisting of the amino acid sequence shown by SEQ ID NO: 2, or an amino acid sequence of a non-human mammal, which corresponds to SEQ ID NO: 2, wherein one or several amino acid residues are substituted, deleted, inserted or added.

Since an antibody that recognizes the polypeptide of the aforementioned (1) or (2) can bind to DPP-4 and inhibit its function, it can be an effective prophylactic and/or therapeutic means for diabetes. That is, administration of the antibody is expected to show a therapeutic effect for the patients who developed diabetes, and a prophylactic effect for the subject having a risk of developing diabetes. Moreover, since the antibody of the present invention is originally present in the body, the risk of side effects is seldom conceived.

The antibody of the present invention includes natural antibodies such as polyclonal antibody, monoclonal antibody and the like, chimeric antibody that can be produced using transgenic mouse and gene recombination technique, humanized antibody and single strand antibody, human antibody produced by mouse, phage display and the like introduced with a human antibody-producing gene, fragments of these and the like. The antibody of the present invention is not particularly limited as long as it recognizes each polypeptide of the present invention, and inhibits the function of DPP-4. From the aspects of specificity to DPP-4, a monoclonal antibody is preferable. From the aspects of clinical application to human, the antibody of the present invention is preferably a humanized antibody or human antibody.

The above-mentioned antibody fragment means a region of one part of the aforementioned antibody, and specific examples thereof include F(ab')₂, Fab', Fab, antibody fragments including Fc regions, Fv (variable fragment of antibody), sFv, dsFv (disulphide stabilized Fv), dAb (single domain antibody) and the like (Exp. Opin. Ther. Patents, Vol. 6, No. 5, p. 441-456, 1996).

The above-mentioned humanized antibody refers to an antibody produced by the gene recombination technique to have an antigen recognition site derived from a gene other than human and the rest derived from a human gene. The above-mentioned human antibody refers to a human antibody produced by a transgenic mouse introduced with a human antibody producing gene (e.g., TransChromo Mouse (trade mark)), or an antibody produced based on a human antibody library in which an antibody variable region is expressed by the display technique such as phage display method and the like from a library constructed by randomly combining human B lymphocyte mRNA, and VH gene and VL gene derived from genome.

The class of the antibody is not particularly limited, and the antibody of the present invention encompasses antibodies having any isotype of IgG, IgM, IgA, IgD, IgE and the like. Preferred is IgG or IgM and, in consideration of the easiness of the purification of antibody and the like, more preferred is IgG.

The production method of the antibody is explained below.

Polyclonal antibody and monoclonal antibody can be produced by a method known per se. That is, in the case of a polyclonal antibody, a mammal, for example, mouse, rat, hamster, guinea pig, rabbit, cat, dog, swine, goat, horse, bovine and the like, preferably mouse, rat, hamster, guinea pig, goat, horse or rabbit, is immunized with an immunogen (the polypeptide of the present invention) together with Freund's adjuvant as necessary. In the case of a monoclonal antibody, mouse, rat, hamster and the like are immunized by a similar method.

While the polypeptide of the present invention can be directly used as an immunogen, it is desirably used for immunization as a complex with a polymer compound having a molecular weight of not less than 10,000. Therefore, when the polypeptide of the present invention is used as an immunogen, a complex may be formed with a polymer compound (e.g., carrier protein and the like) by a method known per se. For example, a polypeptide consisting of the amino acid sequence shown by SEQ ID NO: 2 is synthesized according to the method described above, and a complex with a carrier protein such as bovine serum albumin (BSA), rabbit serum albumin (RSA), ovalbumin (OVA), keyhole-limpet hemocyanin (KLH), thyroglobulin (TG), immunoglobulin and the like is formed. The complex can be thereafter used as a preferable immunogen. As the complex, a complex with keyhole-limpet hemocyanin is preferably used.

To form a complex of the aforementioned polypeptide and a carrier protein and the like, 1-2, preferably one amino acid can be added to the polypeptide of the present invention. While the position of amino acid to be added may be any position in the polypeptide and is not particularly limited, the N-terminal or C-terminal of the polypeptide is preferable.

In the formation of a complex, a known method can be applied without limitation as long as the antigenicity of the polypeptide of the present invention can be maintained. For example, a cysteine residue is introduced into the polypeptide of the present invention, and the polypeptide can be bound to the amino group of the aforementioned polymer compound (carrier protein) via an SH group which is the side chain of cysteine (MBS method). In addition, amino groups such as ε amino group, α amino group and the like of the lysine residue of a protein can also be bound to each other (glutaraldehyde method).

Polyclonal antibody can be specifically produced as follows. That is, mouse, rat, hamster, guinea pig, goat, horse or rabbit, preferably goat, horse or rabbit, more preferably rabbit, is immunized by subcutaneous, intramuscular, intravenous, intrafootpad or intraperitoneal injection of an immunogen 1 - several times. Generally, immunization is performed 1 - 5 times every about 1 - 14 days from the initial immunization, and the serum is obtained from the immunized mammal about 1 - 5 days from the final immunization.

While serum per se can also be used as a polyclonal antibody, it is also possible to isolate and/or purify the antibody by ultrafiltration, ammonium sulfate fraction, eugloblin precipitation, caproinic acid method, caprylic acid method, ion exchange chromatography (DEAE or DE52 etc.), affinity column chromatography using an anti-immunoglobulin column or protein A/G column, a column crosslinked with immunogen and the like and use the obtained purified antibody.

Examples of the production method of the monoclonal antibody include the following methods. A hybridoma is prepared from antibody-producing cells obtained from the above-mentioned immunized animal and myeloma cells without an autoantibody producing ability, and the hybridoma is cloned. That is, a clone that produces a monoclonal antibody showing specific affinity for the peptide of the present invention used for immunizing the mammal and does not show intersection reactivity with a carrier protein is selected using the culture supernatant of hybridoma as a sample and by an immunological method. Then, an antibody can be produced from the culture supernatant and the like of the hybridoma by a method known per se.

To be specific, a monoclonal antibody can be produced as follows. That is, mouse, rat or hamster (including transgenic animal generated to produce an antibody derived from other animal such as human antibody producing transgenic mouse) is immunized by subcutaneous, intramuscular, intravenous, intrafootpad or intraperitoneal injection 1 - several times or transplantation of an immunogen. Generally, immunization is performed 1 - 4 times every about 1 - 14 days from the initial immunization, and the antibody producing cell is obtained from the spleen etc. of the immunized mammal about 1 - 5 days from the final immunization.

A hybridoma (fusion cell) that secretes monoclonal antibody can be prepared by the method of Köhler and Milstein et al. (Nature, Vol. 256, p. 495-497, 1975) and a modified method according thereto. That is, hybridoma is obtained by cell fusion of antibody producing cells contained in the spleen, lymph node, bone marrow, tonsil etc., preferably spleen, obtained from a mammal immunized as mentioned above, and myeloma cells free of an autoantibody producing ability, which are derived from a mammal preferably mouse, rat, guinea pig, hamster, rabbit, human and the like, more preferably mouse, rat or human.

Examples of the myeloma cells to be used for cell fusion include myeloma P3/X63-AG8.653 (653; ATCC No.CRL1580), P3/NSI/1-Ag4-1 (NS-1), P3/X63-Ag8.U1 (P3U1), SP2/0-Ag14 (Sp2/0, Sp2), PAI, F0 or BW5147 derived from mouse, myeloma 210RCY3-Ag.2.3. derived from rat, myeloma U-266AR1, GM1500-6TG-A1-2, UC729-6, CEM-AGR, D1R11 or CEM-T15 derived from human.

A hybridoma producing a monoclonal antibody can be screened for by culturing the obtained hybridoma in, for example, a microtiter plate, measuring the reactivity of the culture supernatant, in the well showing growth, to the polypeptide of the present invention used for the aforementioned immunization and the reactivity of the aforementioned supernatant to a carrier protein, by, for example, an immunoassay method such as ELISA and the like, and comparing them.

A hybridoma cloned by screening is cultured in a medium (e.g., DMEM containing 10% bovine calf serum). The centrifuged supernatant of the culture medium can be used as a monoclonal antibody solution. By injecting the hybridoma into the abdominal cavity of an animal from which the hybridoma is derived, ascites is produced in the animal, and the ascites obtained from the animal can be used as a monoclonal antibody solution. Monoclonal antibody is preferably isolated and/or purified by a method similar to that of the aforementioned polyclonal antibody.

Chimeric antibody can be produced by reference to, for example, "Experimental Medicine (extra edition), Vol. 6, No. 10, 1988", JP-B-3-73280 and the like, humanized antibody can be produced by reference to, for example, JP-A-4-506458, JP-A-62-296890 and the like, and human antibody can be produced by reference to, for example, "Nature Genetics, Vol. 15, p. 146-156, 1997", "Nature Genetics, Vol. 7, p. 13-21, 1994", JP-A-4-504365, WO 94/25585, "NIKKEI Science, June, pages 40-50, 1995", "Nature, Vol. 368, p. 856-859, 1994", JP-A-6-500233 and the like.

An antibody by phage display can be produced by, for example, recovering and concentrating a phage having affinity for antigen by biopanning from a phage library prepared for screening human antibody, whereby antibody such as Fab and the like, and the like can be obtained easily. In this case, the antibody library is preferably screened using the polypeptide of the present invention as an antigen. Refer to "Science, 228: 4075 p. 1315-1317 (1985)", "Nature, 348: p. 552-554 (1990)", "Curr. Protein Pept. Sci., Sep; 1(2): 155-169 (2000)", WO 01/062907 and the like for a preferable antibody library and screening method of antibody. An antibody can be prepared using an antibody fragment obtained thereby or utilizing DNA that the phage has.

The polypeptide of the present invention is obtained by investigating the amino acid sequences in the periphery of the active hole of DPP-4. Therefore, the antibody of the present invention recognizes DPP-4, inhibits the function thereof (GLP-1 degradation), and is expected to show an insulin secretion promoting effect. Consequently, diabetes can be prevented and treated.

The amount of the aforementioned antibody in the prophylactic or therapeutic agent of the present invention is not particularly limited as long as the above-mentioned effect can be afforded. It is generally 0.001 - 90 wt%, preferably 0.005 - 50 wt%, more preferably 0.01 - 10 wt%, of the whole prophylactic or therapeutic agent of the present invention.

The prophylactic or therapeutic agent of the present invention may contain a pharmaceutically acceptable carrier besides the aforementioned antibody as the active ingredient. As such carrier, a carrier generally used in the pharmaceutical field can be used. Examples thereof include, but are not limited to, excipients such as sucrose, starch, mannit, sorbit, lactose, glucose, calcium phosphate, calcium carbonate and the like, preservatives such as sodium benzoate, sodium bisulfite, methylparaben, propylparaben and the like, stabilizers such as citric acid, sodium citrate, acetic acid and the like, suspensions such as methylcellulose, polyvinylpyrrolidone, aluminum stearate and the like, dispersing agents such as surfactant and the like, diluents such as water, saline and the like, basewaxes such as glycerol, polyethylene glycol and the like, and the like.

Examples of the administration dosage form of the prophylactic or therapeutic agent of the present invention include, but are not limited to, liquid, injection preparation and the like. The dosage form of the prophylactic or therapeutic agent of the present invention may be a controlled-release preparation such as an immediate-release preparation, a sustained-release preparation and the like. Since antibody is generally soluble in aqueous solvents, it is easily absorbed in any of the above-mentioned dosage forms. It is also possible to further increase the solubility of the antibody by a method known per se.

The prophylactic or therapeutic agent of the present invention that can be used for the prophylaxis, treatment or alleviation of diabetes can be produced using the above-mentioned antibody as an active ingredient according to a means known per se as a preparation production method.

For example, the prophylactic or therapeutic agent of the present invention preferable for systemic administration can be produced by dissolving an effective amount of the antibody of the present invention in an aqueous or non-aqueous isotonic aseptic injection (e.g., injection preparation). It may be produced by freeze-drying the antibody of the present invention (e.g., freeze-dry preparation) and dissolving same in an aqueous or non-aqueous isotonic aseptic dilution solution. The prophylactic or therapeutic agent of the present invention preferable for topical administration can be produced by dissolving the antibody of the present invention in a dilution solution such as water and saline (e.g., liquid). The liquid can also be used for inhalation therapy into the bronchus, lung and the like by using a sprayer. These agents may contain antioxidant, buffer, bacteriostatic agent, isotonicity agent and the like. These prophylactic or therapeutic agents of the present invention can be sealed in a unit dose or plural dose container such as ampoule or vial.

While the dose of the prophylactic or therapeutic agent of the present invention can be appropriately determined according to the activity, kind or the amount of the antibody contained as the active ingredient, subject of administration, administration route, age and body weight of the subject of administration, and the like, for example, the daily dose (effective amount) for an adult (body weight 60 kg) in the amount of the antibody is 0.1 mg - 1000 mg, preferably 0.1 mg-500 mg, more preferably 0.1 mg - 300 mg. The prophylactic or therapeutic agent of the present invention can be administered in one to several portions per day as necessary, and can also be administered in several days.

The prophylactic or therapeutic agent of the present invention can be used in combination with known prophylactic or therapeutic agents for diabetes. Examples of such known prophylactic or therapeutic agents for diabetes include DPP-4 inhibitors such as sitagliptin, vildagliptin, alogliptin, linagliptin, teneligliptin, anagliptin and the like, anti-diabetes drugs such as insulin, tolbutamide, glyclopyramide, glibenclamide, metformin, epalrestat, voglibose, acarbose, troglitazone and the like, and the like. Only one kind may be used for combination, or plural kinds thereof may be used for combination. In the present specification, "combined use" means that the prophylactic or therapeutic agent of the present invention and known prophylactic or therapeutic agents for diabetes are used in combination, and the use form thereof is not particularly limited. For example, it includes both administration of a pharmaceutical composition containing the prophylactic or therapeutic agent of the present invention and known prophylactic or therapeutic agents for diabetes, and administration thereof, which are separately formulated without mixing, simultaneously or in a staggered manner.

### Examples

The present invention is explained in more detail in the following by referring to Examples. It is needless to say that the present invention is not limited thereby.

### Vaccine design and syntheses

To produce a neutralizing antibody, we designed a site in the N terminal sequence of DPP-4 (E1; SEQ ID NO: 5), and two sequences (E2; SEQ ID NO: 6, E3; SEQ ID NO: 4) in the periphery of the active hole of DPP-4. Keyhole limpet hemocyanin (KLH) (Wako Pure Chemical Industries), which can present a variety of T cell epitopes necessary for increasing the immunogenicity and helps breaking the tolerance toward a peptide sequence as a vaccine was conjugated to the N terminal of 3 candidate peptides. The high quality synthetic peptides (>98% purity) were obtained, and purified by reverse-phase HPLC (Peptide Institute Inc.).

### Animal study and Immunization

Eight-week-old male C57/BL6J mice and six-week-old male db/db mice were purchased (Oriental Yeast Company) and raised in a temperature- and light cycle-controlled facility with free access to food and water. In high-fat diet mouse model, at 8 age week of C57/BL6J mice fed with high-fat diet (HFD60:18.2% protein, 62.2% fat and 19.6% carbohydrate, Oriental Yeast Company). The peptide solution were mixed with an equal volume of Freund's adjuvant (CFA/IFA, Wako Pure Chemical Industries) prior to immunization. Groups of mice (n=6) were injected subcutaneously on days 0, 14, 28, 84 and 119 with either 2 µg or 20 µg of DPP-4 peptide. The control groups of mice were injected with equal quality of KLH mixed with equal volume of Freund's adjuvant. Serum was collected from the tail vein, and antibody titer against the immunizing peptide was determined by ELISA after each boost.

### ELISA

ELISA plate was coated with each candidate peptide at 5 mg/ml overnight at 4°C, which had been conjugated to bovine serum albumin (BSA Peptide Institute Inc.) as a carrier. All wells were blocked using PBS containing 3% skim milk. The serum was diluted over a range from 100 to 325000-fold with block buffer. Following incubation (overnight at 4°C) of the plate and serum and, after washing, the plate was incubated with horseradish peroxidase (HRP)-conjugated antibodies specific for mouse IgG (GE Healthcare) for 3 hours at room temperature. In IgG subclass determination assay, anti-mouse Ig subclass-specific HRP-conjugated antibodies (IgG1, IgG2b and IgG2c) were used. After washing, the peroxidase chromogenic substrate, 3,3'-5,5'-tetramethyl benzidine (TMB; Sigma) was added into ELISA plate wells to develop reaction. All plates were analyzed using a microplate reader (Bio-Rad Inc., Japan) at 450 nm. Half maximal antibody titer was determined by the highest OD450 value in the dilution range of each sample.

### DPP-4 assay

DPP-4 activity was assessed in the plasma 15 min after the meal challenge on day 28, day 42 and day 56. To assess DPP-4 activity, 5 µl serum was mixed with DPP-4-GloTM Reagent solution (DPP-4Glo protease assay; Promega, Madison, WI) and an assay buffer (100 mM HEPES, pH 7.6, 0.1 mg/ml bovine serum albumin) in a total volume of 60 µl. To assess neutralization of anti-DPP-4 antibody, 1 µl of serum was incubated with recombinant DPP-4 (R&D Systems, Inc.) for 1 hour at 4°C, followed by addition of DPP-4-GloTM Reagent solution as described above. The released luminescence was recorded every 1min for 30 min using a SpectraFluor. The data are expressed as % inhibition calculated as follows:
% Inhibition = 100 (1 -(Vi/Vc)), wherein Vi is the rate of reaction of Immunized sample and Vc is the rate of reaction of control sample. In plasma DPP-4 antigen measurement, the serum was incubated in ELISA plate (R&D Systems, Inc.) according to the method attached to the mouse DPP-4 ELISA kit, and read on a microplate reader (Bio-Rad Inc., Japan).

### Western blot assay

The recombinant DPP-4 and BSA-DPP-4 conjugate were separated electrophoretically by sodium dodecylsulfate polyacrylamide gel electrophoresis and blotted onto polyvinylidene difluoride membranes. The blots were incubated with serum and commercial anti-DPP-4 antibody (CD26 (T-19): sc-7044, Santa Cruz Biotechnology, Inc.) respectively. After incubation of HRP-conjugated antibodies specific for mouse IgG, the membranes were visualized and electrochemiluminescence signals were quantified.

### Meal tolerance test and plasma parameters

The mice were fasted overnight, a meal (14% protein, fat 31.5%, CHO 54.5% Ensure H, Meiji, Tokyo, Japan) solution was administered orally at dose of 2 g CHO/kg. The plasma insulin concentration (Mouse ELISA insulin kit, Morinaga, Japan), plasma active GLP-1 (EMD Millipore, Inc. USA) and Blood glucose levels were measured at each indicated time point. Blood glucose was determined from tail bleeds taken 0, 30, 60, 90, and 120 min after meal challenge by glucometer. The blood glucose excursion profile from t= 0 to t =120 min was used to integrate an area under the curve (AUC0-2h). Percent inhibition values for each treatment were generated from the AUC data normalized to the meal challenged lean controls.

### Pancreatic insulin content and tissue analysis

The whole pancreas was isolated immediately so that fat and other pancreatic tissues would not attach. In insulin content assay, after measuring the wet weight, the isolated pancreas was frozen in liquid nitrogen and stored at -80°C until use. The pancreas was thawed and homogenized with PBS containing a protease inhibitor cocktail. The homogenates were centrifuged (1000 g, 10 min, 4°C) and the supernatant was prepared for insulin assay (Mouse ELISA insulin kit, Morinaga). In immunohistochemistry analysis, the isolated pancreas was fixed in 4% paraformaldehyde for 24 hr and embedded in paraffin, and cut into 4 µm sections. The sections were reacted with primary antibody (guinea pig anti-insulin antibody, Dako) and the secondary antibody (biotinylated anti-guinea pig IgG antibody). Slides were finally counterstained with haematoxylin and subjected to microscopic observation. In histological examination assay, the jejunum, liver and kidney were dissected, fixed in 4% paraformaldehyde overnight and embedded in paraffin. The 4 µm sections of liver and kidney were stained with Masson trichrome.

### T cell proliferation assay

The immunized mice were sacrificed at the end of the experiment period, splenocytes (10⁶ cell/well) were cultured in RPMI 1640 medium and stimulated with candidate peptide, KLH and phytohemagglutinin (PHA) (Wako Pure Chemical Industries) at 10 µg/ml. After incubation at 37°C for 48 hr, 1 µCi [3H] thymidine (Perkin Elmer) was added to each well and the plates were incubated for another 8 hr. The [3H] thymidine uptake was determined using MicroBeta 1450 TriLux scintillation counter (Wallac Oy).

### ELISPOT assay

The 96-well ELISPOT plates were coated with anti-mouse IFN-α antibody and anti-mouse IL-4 capture antibody respectively at 4°C overnight. After incubation, the plates were washed with PBS containing 0.05% Tween 20 (PBS-T) solution and blocked with PBS containing 1% BSA and 5% sucrose. Then, the splenocytes from immunized mice suspensions were seeded in wells (10⁶ cell/well), and restimulated with 10 µg/ml candidate peptide, KLH and PHA at 37°C for 48 hr. After incubation, the plates were washed with PBS-T. Then the wells were incubated with biotinylated anti-mouse IFN-α antibody, or biotinylated anti-mouse IL-4 antibody overnight at 4°C and washed with PBS-T. The streptavidin-AP was added into each well and incubated for 2 hr at room temperature. After washing with PBS-T, the plates were incubated with BCIP/NBT solution for 30 min at room temperature. Lastly, the plates were rinsed with water, air-dried at room temperature, and colored spots were counted using a dissecting microscope (Olympus).

### Statistical analysis

All data are expressed as mean±SEM. Statistical analyses were performed using Prism GraphPad version 5.01 (GraphPad Software Inc.). A statistical difference was considered significant when P<0.05.

### Example 1 Selection and screening of appropriate antigen sequence for DPP-4 vaccine

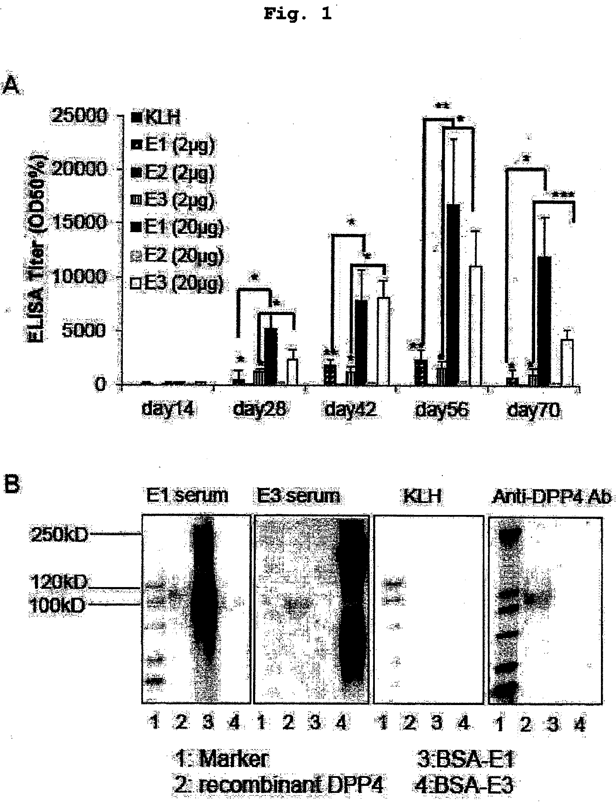

Based on the three-dimensional structure, 3 peptides: a site in the N terminal sequence of DPP-4 protein (E1; SEQ ID NO: 5), and two other sequences (E2; SEQ ID NO: 6, E3; SEQ ID NO: 4) were designed. Since the induced-antibody could overlap the active hole of DPP-4, it was expected to function as a DPP-4 neutralizing antibody. Three candidate peptides (E1, E2, E3) were conjugated to KLH, and injected 3 times to male C57BL/6J mice (8-week-old, n=6/group) at a low dose (2 µg peptide/mouse) and a high dose (20 g peptide/mouse) at 2-week-intervals (Fig. 1A). The antibody titer (shown in half maximal) to DPP-4 did not increase for 14 days after the first immunization. However, in the mice immunized with E1 or E3 vaccine (hereinafter sometimes to be described as E1 vaccine group, E3 vaccine group, respectively), the antibody titer strikingly rose on day 28 in a dose dependent manner, further increased on days 42 and 56, and gradually decreased on day 70. In the mice vaccinated only with KLH or E2 vaccine (hereinafter sometimes to be described as KLH vaccine group, E2 vaccine group, respectively), the antibody did not increase. These results indicate that an average half-life of anti-DPP-4 antibody in E1 and E3 vaccine groups was about 42 days, which is higher than the half-life of all existent DPP-4 inhibitory compounds. On day 56 from the start of the immunization, the antibody titer in high dose vaccine mice (20 µg peptide/mouse) was about 6 times higher than that in the low dose vaccine mice (2 µg peptide/mouse). Whether antibodies induced with E1 or E3 vaccine can recognize DPP-4 antigen and bind thereto was evaluated. From the results of Western blot, the antibodies derived from the serum of E1 or E3 vaccine group recognized recombinant DPP-4 protein, similar to BSA conjugate E1 or E3 (Fig. 1B).

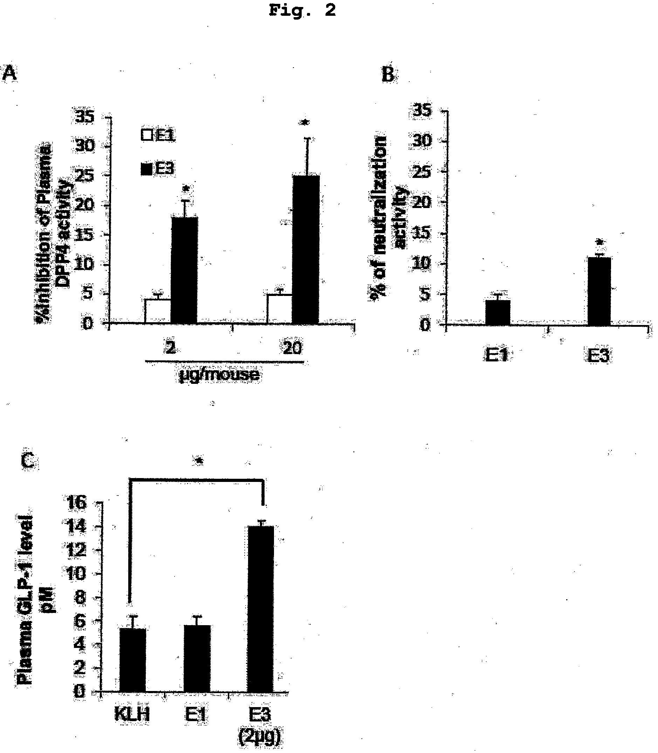

To further evaluate the inhibitory function of anti-DPP-4 antibody induced with E1 or E3 vaccine, the inhibition rate of plasma DPP-4 activity was measured in E1 and E3 groups on day 28, day 42 and day 56 after immunization. E3 vaccine (20 µg/mouse, 2 µg/mouse)-induced antibodies showed a decrease in the plasma DPP-4 activity (25% inhibition, 18% inhibition, respectively, P<0.05) in a vaccine dose-dependent manner (Fig. 2A). However, E1 vaccine-induced antibodies did not show a decrease in the plasma DPP-4 activity. In addition, the DPP-4 neutralization activity of anti-DPP-4 antibodies induced by vaccine to DPP-4 was evaluated by in vitro neutralization assay. The serum of derived from mouse immunized with E3 vaccine showed DPP-4 neutralization activity (13% inhibition, P<0.05) but it scarcely increased in the E1 vaccine group (Fig. 2B). Furthermore, along with increasing antibody titer of E3 vaccine-induced antibody, plasma DPP-4 activity inhibitory rate increased in a time-dependent manner (day 28, day 42, day 56) (Fig. 3A), and neutralization activity of the antibody increased (Fig. 3B). Thus, these results indicate that the E3 vaccine-induced antibodies effectively inhibited the DPP-4 activity in vivo and in vitro.

To confirm the DPP-4 activity inhibitory action, the plasma GLP-1 level of KLH, E1 or E3 vaccine-immunized mice was measured. It was well known that the plasma GLP-1 level reaches the maximum in 5 minutes after oral glucose administration. As a result of the measurement, the plasma GLP-1 level markedly increased only in the E3 vaccine group; however, the E1 vaccine group and KLH vaccine group showed a low level similar to the lean mouse (Fig. 2C).

### Example 2 Improvement in insulin resistance by E3 vaccine in high-fat diet mouse

Since secretion of GLP-1 also by composite nutrient, which is similar to that by glucose, has been reported (Yamazaki K et al., J Pharmacol Sci. 2007 May; 104(1): 29-38), the effect of E3 vaccine in glucose metabolism was valuated by performing a meal tolerance test. As a result, E3 vaccine-immunized male mice (8-week-old, n=6/group) did not show a decrease in the glucose level or insulin level under normal diet conditions, as compared to KLH vaccine-immunized control mouse (Fig. 4A, B). These results suggest that E3 vaccine does not induce hypoglycemia in mouse under general diet conditions.

To further evaluate the effect of E3 vaccine, from the start of the immunization, C57BL/6J mouse (8-week-old, n=6/group) was bred on a high-fat diet (60% fat) (Fig. 4C), an oral meal tolerance test (MTT) was performed on day 105, and the impaired glucose tolerance-improving effect of the E3 vaccine was examined. After E3 vaccine immunization, the plasma glucose level of the mouse immunized with a high dose (20 µg peptide/mouse) was lower than that of the KLH vaccine group (Fig. 4D, E). In the mouse immunized with a high dose E3 vaccine, a clear decrease in the insulin level, which increased in the plasma of the high-fat diet mouse, could be confirmed (Fig. 5A). Furthermore, as compared to the KLH vaccine group, significant improvement in HOMA-IR, which is a useful parameter for the evaluation of insulin sensitivity, was confirmed in the mouse immunized with a high dose E3 vaccine (Fig. 5B). In an intraperitoneal insulin tolerance test (IPITT), high dose E3 vaccine remarkably improved the decrease rate of blood glucose of high-fat diet mouse after insulin administration (Fig. 5C), and improvement of insulin resistance was found. The plasma GLP-1 level was measured then, and the plasma GLP-1 level markedly increased in a vaccine dose-dependent manner (Fig. 5D).

The increase in the antibody titer was maintained for about 3 months by 3 times of the administration of E3 vaccine. It was further confirmed that administration of the E3 vaccine again reincreases the antibody titer due to a booster effect (Fig. 6A). In the high-fat diet mouse administered with E3 vaccine, the plasma DPP-4 level decreased (20% decrease vs KLH vaccine group) (Fig. 6B).

Remarkable changes in the body weight increase and diet consumption were not present between the E3 vaccine group and the KLH vaccine group (Fig. 6C, D).

### Example 3 Evaluation of high-fat diet-induced initial type 2 diabetic model mouse

To further investigate the effectiveness of E3 vaccine in diabetes model, the high-fat diet-induced initial type 2 diabetic model mouse reported before (Guim K et al., Diabetes December 2004 53:S225-S232) was used. The mouse (8-week-old, n=6/group) was fed with a high-fat diet for 5 weeks before the initial E3 vaccine (20 µg/mouse) administration (Fig. 7A). The DPP-4 activity inhibitory rate increased in the E3 vaccine group as compared to the KLH vaccine group (22%, p<0.05). In a meal tolerance test on day 56, E3 vaccine improved glucose excursion (Fig. 7B, C). These results suggest that E3 vaccine improves not only insulin resistance, but initial symptoms of type 2 diabetes.

### Example 4 Delayed onset of diabetes in db/db mouse

To examine the effect of E3 vaccine on the progress of diabetes, young db/db mouse (6-week-old, n=5/group) was immunized with E3 vaccine (20 µg/mouse). A meal tolerance test was performed on day 28 from the start of the immunization, and a decrease in the postprandial blood glucose level was confirmed (10% decrease in AUC₀₋₂ₕ) (Fig. 8A, B). To evaluate the blood glucose control level, plasma insulin level and pancreatic insulin content were measured. After oral meal challenge, the plasma insulin level increased in the E3 vaccine group as compared to the KLH vaccine group (5.18 ng/ml E3 vaccine, 3.77 ng/ml KLH) (Fig. 8C). Similarly, E3 vaccine immunization caused a marked increase in the pancreatic insulin content (12.71 ng/ml E3 vaccine, 10.81 ng/ml KLH) (Fig. 8D). These results suggest that E3 vaccine not only increases insulin secretion but also improves proliferation or replication of β cells in the pancreas. As a result of a morphological test, E3 vaccine was suggested to suppress decrease and destruction of P cell mass as compared to the KLH vaccine group (Fig. 8E).

To examine the mechanism of E3 vaccine relative to insulin secretion in db/db mouse, the plasma active GLP-1 level and plasma DPP-4 level were examined (Fig. 9A, B). In the E3 vaccine group, plasma DPP-4 was neutralized and, on the other hand, plasma active GLP-1 level increased as compared to the KLH vaccine group, (38% increase vs KLH vaccine group, p<0.05). These results suggest that E3 vaccine-induced antibody increased endogenous GLP-1 level that stimulates pancreatic insulin secretion, and finally decreases the blood glucose level.

### Example 5 Evaluation of T cell activation in immunization

To analyze T cell response in the mouse immunized with E3 vaccine, IgG subclass ELISA assay, T cell proliferation assay and ELISPOT assay were used to examine T cell activation in mouse after immunization. In the IgG subclass ELISA assay, the proportion of IgG1 anti-DPP-4 antibody to IgG2b anti-DPP-4 antibody in the E3 vaccine group was 1 or more (1:500 dilution), which shows that E3 vaccine mainly induces Th2 type antibody relative to DPP-4 (Fig. 10A). To further detect Th1 type and Th2 type in T cell response, production of IFN-α (Th1) and IL-4 (Th2) in splenocytes was examined by ELISPOT assay. While stimulation by KLH induced production of IFN-α and IL-4, and further remarkably increased the number of splenocytes that produce IL-4, DPP-4 peptide and the control showed no induction (Fig. 10C, D). In the T cell proliferation assay, the splenocytes derived from mouse immunized with E3 vaccine did not induce marked T cell proliferation even after stimulation with E3 peptide (Fig. 10B). These results suggest that KLH has an appropriate T cell epitope for inducing activation of T cells, but DPP-4 does not. Furthermore, after stimulation with E3 vaccine, most of the T cells promoted antibody production, and were differentiated into Th2 type that decreases the risk of induction of autoimmune responses.

In addition, immune modulation damage in tissues such as jejunum, liver, kidney and the like expressing endothelial DPP-4 at a high level was evaluated. After immunization of mouse with E3 vaccine, clear tissue damage or leukocyte accumulation was not found when compared to the control group (Fig. 11). The results suggest that E3 vaccine did not stimulate antigen-antibody reaction.

### Industrial Applicability

Using the partial amino acid sequence of DPP-4 of the present invention as a vaccine, a neutralizing antibody that inhibits DPP-4 activity can be induced, degradation of GLP-1 can be inhibited by the antibody, and insulin secretion can be improved. Since the antibody induced by the vaccine has a long half-life, it does not require frequent administration unlike conventional therapeutic drugs for diabetes.

This application is based on a patent application No. 2013-183390 filed in Japan (filing date: September 4, 2013), the contents of which are incorporated in full herein.

## Claims

1. A vaccine for the prophylaxis or treatment of diabetes, comprising any of the following substances (1) - (3):
(1) a polypeptide comprising the amino acid sequence shown by SEQ ID NO: 2 or an amino acid sequence of a non-human mammal, which corresponds to SEQ ID NO: 2;
(2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added; and
(3) an expression vector capable of expressing the polypeptide of said (1) or (2).

2. The vaccine according to claim 1, comprising a carrier protein.

3. The vaccine according to claim 1 or 2, which comprises an adjuvant.

4. A prophylactic or therapeutic agent for diabetes, comprising an antibody recognizing the polypeptide of the following (1) or (2), and inhibiting the function of DPP-4:
(1) a polypeptide comprising the amino acid sequence shown by SEQ ID NO: 2 or an amino acid sequence of a non-human mammal, which corresponds to SEQ ID NO: 2; and
(2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added.

5. A method for the prophylaxis or treatment of diabetes, comprising administering an effective amount of any of the following substances (1) - (3) to a subject:
(1) a polypeptide comprising the amino acid sequence shown by SEQ ID NO: 2 or an amino acid sequence of a non-human mammal, which corresponds to SEQ ID NO: 2;
(2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added; and
(3) an expression vector capable of expressing the polypeptide of said (1) or (2).

6. The method according to claim 5, comprising administering a carrier protein.

7. The method according to claim 5 or 6, comprising administering an adjuvant.

8. A method for the prophylaxis or treatment of diabetes, comprising administering an effective amount of an antibody recognizing the polypeptide of the following (1) or (2), and inhibiting the function of DPP-4 to a subject:
(1) a polypeptide comprising the amino acid sequence shown by SEQ ID NO: 2 or an amino acid sequence of a non-human mammal, which corresponds to SEQ ID NO: 2; and
(2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added.

9. Any of the following substances (1) - (3) for use for a method for the prophylaxis or treatment of diabetes:
(1) a polypeptide comprising the amino acid sequence shown by SEQ ID NO: 2 or an amino acid sequence of a non-human mammal, which corresponds to SEQ ID NO: 2;
(2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added; and
(3) an expression vector capable of expressing the polypeptide of said (1) or (2).

10. The substance according to claim 9, comprising a carrier protein.

11. The substance according to claim 9 or 10, comprising an adjuvant.

12. An antibody recognizing the polypeptide of the following (1) or (2), and inhibiting the function of DPP-4, which is for use for a method for the prophylaxis or treatment of diabetes:
(1) a polypeptide comprising the amino acid sequence shown by SEQ ID NO: 2 or an amino acid sequence of a non-human mammal, which corresponds to SEQ ID NO: 2; and
(2) a polypeptide comprising the amino acid sequence shown by SEQ ID NO: 2 or an amino acid sequence of a non-human mammal, which corresponds to SEQ ID NO: 2 and in which one or several amino acid residues are substituted, deleted, inserted or added.

13. A polypeptide consisting of the amino acid sequence shown by SEQ ID NO: 2.
